# EUROPÄISCHE PATENTSCHRIFT

(11) **EP 0 835 862 B1**
(45) Veröffentlichungstag und Bekanntmachung des Hinweises auf die Patenterteilung: **21.03.2001**
(21) Anmeldenummer: 97116836.4
(22) Anmeldetag: 27.09.1997
(51) Int. Cl.: C07C 69/34, C07C 69/52, A61K 7/48, C07C 67/08

(54) **Polyglycerinpartialester von Fettsäuren und mehrfunktionellen Carbonsäuren, deren Herstellung und Verwendung**
Partial esters of polyglycerine with fatty acids and polyfunctional carboxylic acids, their preparation and use
Esters partiels du polyglycérole avec des acides gras et des acides carboniques polyfonctionnels, leur préparation et leur utilisation

(30) Priorität: 09.10.1996 DE 19641604
(43) Veröffentlichungstag der Anmeldung: 15.04.1998
(73) Patentinhaber: Goldschmidt AG, 45127 Essen (DE)
(72) Erfinder: Grüning, Burghard, Dr., 45134 Essen (DE); Hameyer, Peter, 45138 Essen (DE); Metzelaars, Josef, 45355 Essen (DE); Weitemeyer, Christian, Dr., 45134 Essen (DE)

(56) Entgegenhaltungen:
- EP-A- 0 051 148
- WO-A-90/05714
- WO-A-92/00269
- DE-A- 4 012 693

## Beschreibung

Die Erfindung betrifft Polyglycerinpartialester von Fettsäuren und mehrfunktionellen Carbonsäuren, erhältlich durch Veresterung eines Polyglyceringemisches mit gesättigten oder ungesättigten, linearen oder verzweigten Fettsäuren mit 12 bis 22 C-Atomen und Dimerfettsäuren, die durch katalytische Dimerisierung ungesättigter Fettsäuren mit 12 bis 22 C-Atomen erhalten wurden und eine mittlere Funktionalität von 2 bis 2,4 aufweisen, wobei der Veresterungsgrad der Polyglycerinmischung zwischen 30 und 75 % liegt, deren Herstellung und Verwendung als W/O-Emulgatoren in kosmetischen oder pharmazeutischen Zubereitungen sowie als Hilfsmittel zur Dispergierung von anorganischen Mikropigmenten in Öldispersionen.

An W/O-Emulgatoren, die auf nativen Rohstoffen basieren, besteht aus ökologischen Gründen sowohl bei den Produzenten als auch bei den Konsumenten von Emulsionspräparaten ein erhebliches Interesse. Aus diesem Grunde finden, trotz ihrer mittelmäßigen Leistungsfähigkeit, Partialester aus Polyalkoholen, wie Glycerin, Polyglycerin, Sorbitol oder Methylglycosid, und Fettsäuren, wie Öl- oder Isostearinsäure, nach wie vor eine vielfältige Verwendung.

Dieser Emulgatortyp ist z. B. für fließfähige Emulsionen (Lotionen) und für Cremes mit hohem Gehalt an nativen Triglyceriden nicht geeignet. Die den Stabilitätsanforderungen des Marktes entsprechenden Cremes (Temperaturbelastbarkeit von -15 bis +45 °C, teilweise von -25 bis +50 °C) enthalten an lipidartigen Komponenten überwiegend Paraffinöle und Fettsäureester von Monoalkanolen (MG < 500); diese haben günstigere technologische Eigenschaften als die höhermolekularen Triglyceride. Zur Stabilisierung werden dennoch relativ hohe Konzentrationen an viskositätserhöhenden Wachsen (≥ 3 %) benötigt, die sich negativ auf die Applikationseigenschaften auswirken, da sie ein unerwünschtes klebrig-fettiges Gefühl auf der Haut erzeugen.

Koemulgatoren, insbesondere Ethylenoxidaddukte in Kombination mit Metallseifen, erweitern den Anwendungsbereich lediglich auf paraffinölhaltige Lotionen.

Erheblich bessere Emulgiereigenschaften als die Polyalkohol-Fettsäurepartialester besitzen die Polyglycerinester di- und polymerisierter ungesättigter C₁₈-Fettsäuren. Sie werden aus den Mono- und Diglyceriden von Pflanzenölen, bevorzugt Sojaöl, durch eine mehrstündige thermische Behandlung bei ca. 300 °C bzw. durch die Umesterung eines thermisch polymerisierten Pflanzenöls mit Polyglycerin gewonnen.

Die nach einem analogen Verfahren aus Rizinusöl gebildeten Polyglycerin-Polyricinoleate sind ebenfalls leistungsfähige W/O-Emulgatoren (DE-B-44 09 569). Beide Substanzklassen haben sich wegen ihrer Oxidationsempfindlichkeit und des ausgeprägten fettig-ranzigen Geruchs nicht in kosmetischen oder pharmazeutischen Emulsionspräparaten etablieren können. Verantwortlich dafür sind in erster Linie die massive thermische Belastung während der Herstellung und der ungesättigte Charakter (Jodzahl ca. 100).

Das dem Polyglycerin-Polyricinoleat chemisch verwandte, ebenfalls aus vegetabilischen Rohstoffen herstellbare Polyglycerin-Polyhydroxystearat (Fa. Henkel) hat demgegenüber eine zufriedenstellende sensorische Qualität und besitzt die prinzipielle Befähigung zur Bildung cremeartiger und insbesondere fließfähiger W/O-Emulsionen.

Gemäß DE-A-40 12 693 wurden Ester von Polycarbonsäuren mit Polyhydroxyverbindungen vorgeschlagen; durch Kondensation der Ausgangsverbindungen erhält man Produkte mit überschüssigen Carboxylgruppen, die anschließend neutralisiert werden. Die Reaktionsprodukte eignen sich als O/W-Emulgatoren.

Die Aufgabe der Erfindung hat nun darin bestanden, neue Polyglycerinpartialester zu schaffen, die aus nativen Rohstoffen herstellbar sind und gegenüber dem Polyglycerin-Polyhydroxystearat den zusätzlichen Vorteil einer verbesserten Stabilität, insbesondere einer höheren Gefrier-Tau-Belastbarkeit der damit hergestellten W/O-Emulsionen aufweisen. Diese Verbesserung ist für die Transport- und Lagerfähigkeit der Emulsionspräparate von erheblichem praktischen Interesse.

Durch längere Lagerung bei sehr tiefen Temperaturen oder durch extreme Temperaturschwankungen bei längeren Transportwegen kann sich die ungenügende Emulsionsstabilisierung nämlich durch deutliche Wasserabscheidungen im Emulsionspräparat zeigen oder sogar zur völligen Brechung der Emulsion führen, was durch die erfindungsgemäße Lösung der Aufgabe vermieden wird.

Gegenstand der Erfindung sind Polyglycerinpartialester von gesättigten oder ungesättigten, linearen oder verzweigten Fettsäuren und mehrfunktionellen Carbonsäuren, die erhältlich sind durch Veresterung eines Polyglyceringemisches mit gesättigten oder ungesättigen, linearen oder verzweigten Fettsäuren mit 12 bis 22 C-Atomen und Dimerfettsäuren, die durch katalytische Dimerisierung ungesättigter Fettsäuren mit 12 bis 22 C-Atomen erhalten wurden und eine mittlere Funktionalität von 2 bis 2,4 aufweisen, wobei der Veresterungsgrad des Polyglycerins zwischen 30 und 75 % liegt.

Als gesättigte Fettsäurekoinponenten eignen sich vor allem Laurinsäure, Tridecansäure, Myristinsäure, Palmitinsäure, Margarinsäure, Stearinsäure, Arachinsäure und Behensäure sowie Mischungen hieraus. Natürlich vorkommende Mischungen sind beispielsweise die Kokosfettsäuren, die als Hauptbestandteil Laurinsäure, daneben noch gesättigte C₁₄- bis C₁₈-Fettsäuren und gegebenenfalls in geringen Mengen gesättigte C₈- bis C₁₀-Fettsäuren und ungesättigte Fettsäuren enthalten, sowie Talgfettsäuren, welche im wesentlichen eine Mischung aus Palmitinsäure und Stearinsäure darstellen. Besonders bevorzugt wird eine Mischung, die aus mindestens 40 Gew.-%, vorzugsweise 60 bis 99 Gew.-% Stearinsäure besteht.

Als ungesättigte Fettsäurekomponenten eignen sich monoolefinisch ungesättigte Säuren, beispielsweise Hexadecensäuren, Octadecensäuren, wie Ölsäure (cis-9-Octadecensäure) oder Elaidinsäure (trans-9-Octadecensäure), Eicosensäuren und Docosensäuren, wie Erucasäure (cis-13-Docosensäure) oder Brassidinsäure (trans-13-Docosensäure), mehrfach ungesättigte Fettsäuren, beispielsweise Octadecadiensäuren und Octadecatriensäuren, wie Linolsäure und Linolensäure, sowie Mischungen hieraus. Die besten Ergebnisse erzielt man mit einer Fettsäure oder Fettsäuremischung, die aus mindestens 60 Gew.-%, vorzugsweise 65 bis 99 Gew.-% Ölsäure oder Erucasäure besteht.

Besonders geeignet sind die flüssigen Fettsäuren, Öl-, Ricinol-, Eruca- und Isostearinsäure, die 18 bis 22 Kohlenstoffatome enthalten. Ihre Erstarrungspunkte liegen aufgrund einer Verzweigung oder einer Doppelbindung in der Kohlenwasserstoffkette unter 35 °C. Verwendbar sind weiterhin Fettsäuregemische, die auch wachsartige Komponenten, wie gehärtete Ricinolsäure, enthalten können.

Bei den eingesetzten Dimerfettsäuren handelt es sich bekanntlich um ein Gemisch aus acyclischen und cyclischen Dicarbonsäuren, die durch eine katalysierte Dimerisierung ungesättigter Fettsäuren mit 12 bis 22 C-Atomen erhalten werden.

Zur Herstellung und Verwendung von Dimersäuren und deren physikalische und chemische Eigenschaften wird auf die Veröffentlichung "The Dimer Acids: The chemical und physical properties, reactions and applications", Ed. E.C. Leonard; Humko Sheffield Chemical, 1975, Memphis, Tenn., verwiesen.

Die Dicarbonsäuren können auch in untergeordnetem Ausmaß tri- und mehrfunktionelle Carbonsäuren enthalten. Die Funktionalität des Gemisches sollte im molaren Mittel einen Wert von 2,4 nicht überschreiten.

Besonders geeignet für den erfindungsgemäßen Einsatzzweck als Emulgatoren ist die Verwendung von Dimersäure oder eines Gemisches aus Dimer- und Trimersäure (z. B. Pripol-Typen der Firma Unichema), die aus Pflanzenölen mit hohem Gehalt an ungesättigten C₁₈-Fettsäuren (Öl-, Linol-, Linolensäure) gewonnen wird. Die durch die Verwendung dieser Säuren erzielbaren wesentlich höheren Molekulargewichte bei in etwa gleichbleibender Zahl der freien Hydroxylgruppen im Produkt hat eine wesentlich verbesserte Stabilisierung der Phasengrenzflächen in W/O-Emulsionen zur Folge.

Als Polyglycerine sind insbesondere solche mit einem mittleren Kondensationsgrad von ≥ 2, bevorzugt 3 bis 4, geeignet. Dabei handelt es sich um technische Polyglycerinmischungen, die z. B. durch alkalisch katalysierte Kondensation von Glycerin bei erhöhten Temperaturen erhalten werden, aus denen sich gegebenenfalls durch Destillationsverfahren Fraktionen mit dem erwünschten Kondensationsgrad erhalten lassen. Ebenfalls geeignet sind auch Polyglycerine, die auf anderem Wege, z. B. aus Epichlorhydrin oder Glycidol gewonnen werden.

Besonders geeignete Polyglycerine weisen folgende Oligomerenverteilung auf:

| | |
|---|---|
| Glycerin | 0 bis 30 Gew.-% |
| Diglycerin | 15 bis 40 Gew.-% |
| Triglycerin | 10 bis 55 Gew.-% |
| Tetraglycerin | 2 bis 25 Gew.-% |
| Pentaglycerin und höhere Anteile | 0 bis 15 Gew.-% |

In den erfindungsgemäßen Polyglycerinpartialestern sind die Hydroxylgruppen des Polyglycerins zu 30 bis 75 %, bevorzugt 50 bis 65 %, verestert. Sie werden zunächst bis zu einem Veresterungsgrad von 25 bis 60 %, bevorzugt 35 bis 50 %, mit Fettsäure verestert und in einem zweiten Schritt mit Dicarbonsäuren zu einem Gesamtveresterungsgrad von 30 bis 75 %, bevorzugt 50 bis 65 %, verestert. Durch geeignete Auswahl der hydrophilen und lipophilen Molekülanteile sollte ein HLB-Wert von 3 bis 7 angestrebt werden, um für die Stabilisierung von W/O-Emulsionen günstige Eigenschaften zu erhalten.

Die erfindungsgemäßen Polyglycerinpartialester können in an sich bekannter Weise durch Erhitzen der Reaktionskomponenten und destillativer Abtrennung des entstehenden Reaktionswassers hergestellt werden. Zur Beschleunigung können saure oder basische Katalysatoren, wie Sulfonsäuren, Phosphorsäure oder phosphorige Säure, Lewissäuren, wie Zinnsalze, Alkali- oder Erdalkalimetalloxide oder -hydroxide, -alkoholate oder -salze eingesetzt werden. Der Zusatz eines Katalysators ist aber nicht unbedingt notwendig. Die Polyglycerinpartialester werden bevorzugt in einem zweistufigen Verfahren hergestellt, die wiederum in an sich bekannter Weise durchgeführt werden. Dazu wird im ersten Schritt das Polyglycerin mit der monofunktionellen Fettsäure oder einem Teil der Fettsäure verestert. Nachdem die Fettsäure zum größten Teil oder vollständig umgesetzt ist, wird nachfolgend die mehrfunktionelle Carbonsäure hinzugefügt und die Veresterungsreaktion fortgesetzt. Der fortschreitende Umsatz kann z. B. über das abgetrennte Reaktionswasser, durch Messung der Säurezahl oder durch Infrarotspektroskopie verfolgt werden. Im allgemeinen wird eine Säurezahl im Endprodukt von < 20, bevorzugt < 10 angestrebt. Besonders bevorzugt sind Produkte mit einer Säurezahl < 5.

Die erfindungsgemäßen Polyglycerinester eignen sich zur Stabilisierung von Emulsionen und Dispersionen. Bevorzugt ist ihre Verwendung als Emulgatoren zur Herstellung kosmetischer oder pharmazeutischer Zubereitungen.

In Frage kommende kosmetische Zubereitungen, die durch Verwendung von Öl-in-Wasser- oder Wasser-in-Öl-Emulgatoren eine leicht streichbare Konsistenz erhalten, weil durch diese Emulgatorsysteme sich ein Öl oder ein Fett gut in eine wäßrige Phase bzw. eine wäßrige Phase gut in ein Öl oder ein Fett einarbeiten läßt, sind beispielsweise Cremes, wie Pflegecremes, Babycremes oder Sonnenschutzcremes, Salben, Lotionen oder Schminken. In pharmazeutischen Zubereitungen, wie Salben oder Cremes, benötigt man Öl-in-Wasser- oder Wasser-in-Öl-Emulgatoren zur Applikation von Wirkstoffen.

Als Ölkomponente können eingesetzt werden
- Esteröle, wie Octylpalmitat, Octylstearat, Cetyloctanoat, Capryl/Caprinsäuretriglyceride, Decyloleat, Cetearyloctanoat, Isopropylmyristat, Isopropylpalmitat, C₁₂₋₁₃-Alkylbenzoat, Stearylheptanoat, Dibutyladipate
- Pflanzenöle, wie Weizenkeimöl, Jojobaöl, Olivenöl, Borretschöl, Avocadoöl, Erdnußöl, Mandelöl, Sonnenblumenöl, Walnußöl
- höhere Alkohole, wie Octyldodecanol (oder Guerbet-Alkohole allgemein), Oleylalkohol, und
- paraffinartige (Kohlenwasserstoff)Öle, wie Paraffinöl, Isohexadecane, Polydecene, Vaseline, Paraffinum perliquidum, Squalan.

Die üblichen Zusammensetzungen und Bestandteile sowie übliche Hilfsmittel und Zusatzstoffe, wie Stabilisatoren oder Konservierungsmittel, für derartige kosmetische und pharmazeutische Zubereitungen sind dem Fachmann bekannt und brauchen deshalb hier nicht näher erläutert zu werden.

Die erfindungsgemäß verwendeten Polyglycerinpartialester bewirken gegenüber dem Stand der Technik bei tiefen Temperaturen eine deutlich erhöhte Lagerstabilität der mit ihnen hergestellten kosmetischen und pharmazeutischen Zubereitungen, was bei im Vergleich zu Öl-in-Wasser-Emulsionen schwerer zu stabilisierenden Wasser-in-Öl-Emulsionen besonders bedeutsam ist. Weiterhin benötigt man in der Regel von den Polyglycerinpartialestern geringere Konzentrationen und/oder geringere Mengen von konsistenzgebenden Wachsen in den Zubereitungen, um eine gleiche Wirkung wie mit Mitteln des Standes der Technik zu erzielen.

Die erfindungsgemäßen Polyglycerinester eignen sich weiterhin zur Stabilisierung von Dispersionen verschiedenartiger Pigmente in Ölen. Bevorzugte Pigmente sind anorganische Pigmente. Besonders bevorzugt ist Titandioxid. Als Ölkomponenten können die oben aufgeführten Öle eingesetzt werden.

### Beispiele

### 1. Herstellung

Die Herstellung des Polyglycerinesters erfolgt in zwei Stufen, die in an sich bekannter Weise ablaufen. Zunächst wird Polyglycerin mit Fettsäure verestert. 264 g (0,93 Mol) Isostearinsäure werden mit 100 g technischen Polyglycerins, gekennzeichnet durch eine Hydroxylzahl von 1180, zusammengegeben und unter Stickstoffdurchleitung auf 250 °C erhitzt. Nach drei Stunden Reaktion bei dieser Temperatur beträgt die Säurezahl 6,4. Anschließend wird auf 180 °C abgekühlt, und dem Reaktionsgemisch werden 121 g Dimersäure (Pripol 1025 der Fa. Unichema, bestehend aus ca. 5 % Monocarbonsäure, ca. 75 % difunktioneller und ca. 20 % trifunktioneller Carbonsäure) zugegeben. Der Ansatz wird wieder auf 250 °C erhitzt und bei dieser Temperatur drei Stunden gehalten.

Es wird ein bernsteinfarbenes, zähflüssiges Produkt erhalten, das durch eine Hydroxylzahl von 68 und eine Säurezahl von 1,5 gekennzeichnet ist.

### 2. Anwendungstechnische Überprüfung

### 2.1. W/O-Emulgatoren

Aus den folgenden Beispielrezepturen ist die vielseitige Anwendbarkeit des erfindungsgemäßen Emulgators zur Herstellung von hautpflegenden und -schützenden Emulsionspräparaten zu ersehen.

Sie umfassen:
- Emulsionen mit fließfähiger und pastöser Konsistenz (Lotionen bzw. Cremes),
- Emulsionen, deren Ölphase aus Kohlenwasserstoffen (Paraffinöle), Fettsäuremonoalkylestern oder Fettsäuretriglyceriden besteht,
- Präparate, in deren Öl- bzw. Wasserphase dermatologische Wirkstoffe enthalten sind.

Diese Emulsionen weisen eine außerordentlich hohe Wärme- und Kältebelastbarkeit auf. Sie sind bei 45 °C mindestens 5 Monate und bei 50 ° mindestens 1 Monat stabil; sie überstehen mindestens 5 Gefrier-Tau-Zyklen.

Zum Vergleich wurde der Emulgator Polyglycerylpolyhydroxystearat im Versuch I Rp 6 eingesetzt und die übrigen Komponenten in gleichen Mengen wie in Versuch Rp 5 zur Herstellung einer fließfähigen Emulsion verwandt. Der Vergleichsversuch Rp 6 zeigt nach 1-3 Gefrier-Tau-Zyklen starke Wasserabscheidung.

Ebenfalls zum Vergleich wurde ein aus Veresterung von Polyglycerin mit einem Kondensationsgrad von 3 und ca. 3 Mol Ölsäure hergestellter Emulgator im Versuch II Rp 8 eingesetzt und die übrigen Komponenten in gleichen Mengen wie in Versuch Rp 3 zur Herstellung einer Creme verwandt: Die mit diesem Vergleichsemulgator hergestellte Creme zeigt selbst bei Raumtemperatur nach 3 Monaten und bei 45 °C Lagertemperatur nach 1 Monat Wasserabscheidung und ist nach 3 Gefrier-Tau-Zyklen bei -15°C zersetzt.

### Herstellung der Emulsionen

Die auf 80 bis 85 °C erwärmte Wasserphase wird in die gleich warme Ölphase unter intensivem Rühren eingebracht und unter intensivem Rühren auf 25 bis 30 °C abgekühlt. Falls die durchschnittliche Größe der dispergierten Wassertröpfchen über 1 bis 2 um liegt, muß eine Homogenisierung mit Hilfe einer nach dem Rotor-Stator-Prinzip arbeitenden Emulgiermaschine durchgeführt werden.

### I) Emulsionen mit fließfähiger Konsistenz (Lotionen)

### Ölphasen (Zusammensetzung)

| | Rp 1 | Rp 2 | Rp 3 | Rp 4 | Rp 5 | Vgl Rp 6 |
|---|---|---|---|---|---|---|
| Emulgator | 3,0 % | 3,0 % | 3,0 % | 3,0 % | 3,0 % | 3,0 % |
| Rizinusöl, gehärtet | 0,3 % | 0,3 % | 0,3 % | 0,3 % | 0,3 % | 0,3 % |
| Microwachs (Lunacera M) | 0,2 % | 0,2 % | 0,2 % | 0,2 % | 0,2 % | 0,2 % |
| Paraffinöl (30 mPas) | 10,5 % | 10,0 % | - | - | - | - |
| Isohexadecan | 10,0 % | - | - | - | - | - |
| Isohexadecylpalmitat | - | - | 10,5 % | - | - | - |
| Isooctylpalmitat | - | 13,5 % | - | - | - | - |
| Cetylisooctanoat | - | - | 11,0 % | - | - | - |
| Decyloleat | - | - | - | 10,5 % | - | - |
| Isopropylpalmitat | - | - | - | 11,0 % | - | - |
| C_{8/10}-Triglycerid | - | - | - | - | 23,5 % | 23,5 % |
| Ölphase | 24,0 % | 27,0 % | 25,0 % | 25,0 % | 27,0 % | 27,0 % |

### Wasserphasen (Zusammensetzung)

### 0,3 % Magnesiumsulfat, 2,0 % Glycerin, Wasser ad 100 %

Rp 4 enhält zusätzlich 2 % des Moisturizers Lactil® (Th. Goldschmidt AG, wäßrige Lösung von Natrium-Pyrrolidoncarboxylat, Natriumlactat, Urea und Aminosäuren).

### II) Emulsionen mit pastöser Konsistenz (Cremes)

| Ölphase | Rp 1 | Rp 2 | Rp 3 | Rp 4 | Rp 5 | Rp 6 | Rp 7 | Vgl Rp 8 |
|---|---|---|---|---|---|---|---|---|
| Emulgator | 3,0 % | 3,0 % | 3,0 % | 3,0 % | 3,0 % | 3,0 % | 3,0 % | 3,0 % |
| Rizinusöl, | 0,8 % | 0,8 % | 0,5 % | 0,5 % | 0,8 % | 0,8 % | 0,8 % | 0,5 % |
| gehärtet | | | | | | | | |
| Bienenwachs | 1,2 % | 1,2 % | 0,5 % | 0,5 % | 1,2 % | 1,2 % | 1,2 % | 0,5 % |
| Neo-Heliopan AV | 4,0 % | - | - | - | - | - | - | - |
| Parsol 1789 | 2,0 % | - | - | - | - | - | - | - |
| Alkylbenzoat | 9,5 % | - | - | - | - | - | - | - |
| C_{8/10}-Triglycerid | 9,5 % | - | 16,0 % | - | - | - | - | 16,0 % |
| Avocadoöl | - | - | 10,0 % | - | - | - | - | 10,0 % |
| Paraffinöl | - | - | - | 9,0 % | 10,0 % | - | - | - |
| (30 mPas) | | | | | | | | |
| Cetyloctanoat | - | 10,0 % | - | - | - | - | 11,0 % | - |
| Hexadecylpalmitat | - | 10,0 % | - | - | - | - | - | - |
| Octylpalmitat | - | - | - | 10,0 % | - | 20,0 % | - | - |
| Isohexadecan | - | - | - | - | 10,0 % | - | - | - |
| Octyloctanoat | - | - | - | - | - | - | 7,0 % | - |
| | 30,0 % | 25,0 % | 30,0 % | 23,0 % | 25,0 % | 25,0 % | 23,0 % | 30,0 % |

| Wasserphase | Rp 1 | Rp 2 | Rp 3 | Rp 4 | Rp 5 | Rp 6 | Rp 7 | Vgl Rp 8 |
|---|---|---|---|---|---|---|---|---|
| Magnesiumsulfat | 0,3 % | 0,3 % | 0,3 % | 0,3 % | 0,3 % | 0,3 % | 0,3 % | 0,3 % |
| Glycerin | 2,0 % | 2,0 % | 2,0 % | 2,0 % | 3,0 % | 3,0 % | 3,0 % | 2,0 % |
| Urea | - | 2,0 % | - | - | - | - | - | - |
| Ethanol (96 %ig) | - | - | - | - | 10,0 % | 10,0 % | 10,0 % | - |
| Wasser | 67,7 % | 70,7 % | 67,7 % | 74,7 % | 61,7 % | 61,7 % | 63,7 % | 67,7 % |
| | 70,0 % | 75,0 % | 70,0 % | 77,0 % | 75,0 % | 75,0 % | 77,0 % | 70,0 % |

### 2.2. Verwendung als Dispergierhilfsmittel

Die erfindungsgemäßen Polyglycerinpartialester mit einem HLB-Wert von 3 bis 7 sind als Dispergierhilfsmittel für anorganische Pigmente in Ölkörpern geeignet.

Als anorganische Pigmente kommen alle üblichen Hauptgruppen-Metall- und Übergangsmetalloxide, wie z.B. Eisenoxid, Titandioxid, Zinkoxid, Ceriumoxid, jeweils auch in ihrer mikronisierten Form, in Frage.

### Beispiel

In einer Hochgeschwindigkeitsmühle mit kugelförmigem Mahlmittel werden 72 g Octylpalmitat vorgelegt und unter Rühren 28 g mikrofeines Titandioxid und 6 g des Polyglycerinpartialesters sowie 4 g Aerosil R972 (Fa. Degussa) zugegeben. Nach 30minütigem Mahlen wird eine feinteilige, weiße Dispersion erhalten, aus der sich das TiO₂ auch nach 6wöchiger Lagerzeit nicht wieder absetzt.

### Vergleichsbeispiel

In einer Hochgeschwindigkeitsmühle mit kugelförmigem Mahlmittel werden 72 g Octylpalmitat vorgelegt und unter Rühren 28 g mikrofeines Titandioxid sowie 4 g Aerosil R972 zugegeben. Nach 30minütigem Mahlen wird eine Dispersion erhalten, die ein inhomogenes Aussehen aufweist und aus der sich nach mehrstündigem Stehen ein feinteiliger Bodensatz von TiO₂-Pigment absetzt.

## Patentansprüche

1. Polyglycerinpartialester von gesättigten oder ungesättigten, linearen oder verzweigten Fettsäuren und mehrfunktionellen Carbonsäuren, erhältlich durch Veresterung eines Polyglyceringemisches mit gesättigten oder ungesättigten, linearen oder verzweigten Fettsäuren mit 12 bis 22 C-Atomen und Dimerfettsäuren, die durch katalytische Dimerisierung ungesättigter Fettsäuren mit 12 bis 22 C-Atomen erhalten wurden und eine mittlere Funktionalität von 2 bis 2,4 aufweisen, wobei der Veresterungsgrad der Polyglycerinmischung zwischen 30 und 75 % liegt.

2. Polyglycerinpartialester gemäß Anspruch 1, dadurch gekennzeichnet, daß das Polyglycerin einen mittleren Kondensationsgrad von ≥ 2, bevorzugt von 3 bis 4, aufweist.

3. Verwendung der Polyglycerinpartialester gemäß den Ansprüchen 1 und 2 als W/O-Emulgatoren in kosmetischen oder pharmazeutischen Zubereitungen.

4. Verwendung der Polyglycerinpartialester gemäß den Ansprüchen 1 und 2 als Hilfsmittel zur Dispergierung von anorganischen Mikropigmenten in Öldispersionen.

5. Verfahren zur Herstellung von Polyglycerinpartialestern von gesättigten oder ungesättigten Fettsäuren und mehrfunktionellen Carbonsäuren gemäß Anspruch 1, dadurch gekennzeichnet, daß man technisches Polyglycerin in einer ersten Stufe in an sich bekannter Weise mit der Fettsäure bis zu einem Veresterungsgrad des Polyglycerins von 25 bis 60 % und in einer zweiten Stufe mit der Dimerfettsäure zu einem Gesamtveresterungsgrad von 30 bis 75 % umsetzt.

## Claims

1. A polyglycerol partial ester of a saturated or unsaturated, linear or branched fatty acid and a polyfunctional carboxylic acid, obtainable by esterification of a polyglycerol mixture with saturated or unsaturated, linear or branched fatty acids having 12 to 22 carbon atoms and dimer fatty acids obtained by catalytic dimerization of unsaturated fatty acids having 12 to 22 carbon atoms and have a mean functionality of from 2 to 2.4, the degree of esterification of the polyglycerol mixture being between 30 and 75%.

2. A polyglycerol partial ester as claimed in claim 1, wherein the polyglycerol has a mean degree of condensation of ≥ 2, preferably from 3 to 4.

3. The use of a polyglycerol partial ester as claimed in claims 1 and 2 as W/O emulsifier in cosmetic or pharmaceutical preparations.

4. The use of a polyglycerol partial ester as claimed in claims 1 and 2 as auxiliary for dispersing inorganic micropigments in oil disper-sions.

5. A process for preparing polyglycerol partial esters of saturated or unsaturated fatty acids and polyfunctional carboxylic acids as claimed in claim 1, which comprises reacting technical-grade polyglycerol in a first step in a manner known per se with the fatty acid up to a degree of esterification of the poly-glycerol of from 25 to 60%, and in a second step with the dimer fatty acid to an overall degree of esterification of from 30 to 75%.

## Revendications

1. Esters partiels de polyglycérol d'acides gras, saturés ou insaturés, linéaires ou ramifiés et d'acides carboxyliques polyfonctionnels, pouvant être obtenus par estérification d'un mélange de polyglycérols avec des acides gras saturés ou insaturés, linéaires ou ramifiés comprenant 12 à 22 atomes de carbone et avec des acides gras dimères, qui ont été obtenus par dimérisation catalytique d'acides gras insaturés comprenant 12 à 22 atomes de carbone et présentent une fonctionnalité moyenne de 2 à 2,4, le degré d'estérification du mélange de polyglycérols étant compris entre 30 et 75%.

2. Esters partiels de polyglycérol selon la revendication 1, caractérisés en ce que le polyglycérol présente un degré de condensation moyen ≥ 2, de préférence de 3 à 4.

3. Utilisation des esters partiels de polyglycérol selon les revendications 1 et 2 comme émulsifiants eau dans huile dans des préparations cosmétiques ou pharmaceutiques.

4. Utilisation des esters partiels de polyglycérol selon les revendications 1 et 2 comme adjuvants pour la dispersion de micro-pigments inorganiques dans des dispersions huileuses.

5. Procédé pour la préparation d'esters partiels de polyglycérol d'acides gras saturés ou insaturés et d'acides carboxyliques polyfonctionnels selon la revendication 1, caractérisé en ce qu'on transforme du polyglycérol industriel, dans une première étape, de manière connue en soi, avec l'acide gras jusqu'à un degré d'estérification du polyglycérol de 25 à 60% et, dans une deuxième étape, avec l'acide gras dimère jusqu'à un taux d'estérification totale de 30 à 75%.
